(19) **Europäisches Patentamt**
**European Patent Office**
**Office européen des brevets**

(11) **EP 4 112 747 A1**

(12) **EUROPEAN PATENT APPLICATION**

(43) Date of publication:
**04.01.2023 Bulletin 2023/01**

(21) Application number: **21382569.8**

(22) Date of filing: **28.06.2021**

(51) International Patent Classification (IPC):
***C12Q 1/6886*** *(2018.01)*

(52) Cooperative Patent Classification (CPC):
**C12Q 1/6886;** C12Q 2600/154

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(71) Applicants:
• **Fundación para la Investigación del Hospital Clínico de la Comunidad Valenciana (INCLIVA)**
**46010 Valencia (ES)**

• **Universitat de Valéncia**
**46010 Valencia (ES)**

(72) Inventors:
• **MONTEAGUDO CASTRO, Jose Carlos**
**Valencia (ES)**
• **GONZÁLEZ MUÑOZ, Jose Francisco**
**Valencia (ES)**
• **SÁNCHEZ SENDRA, Beatriz**
**Valencia (ES)**

(74) Representative: **Pons**
**Glorieta Rubén Darío 4**
**28010 Madrid (ES)**

(54) **MOLECULAR TOOLS FOR THE DIAGNOSIS AND PROGNOSIS OF MELANOCYTIC SPITZOID TUMORS**

(57) The present invention relates to novel differentially methylated genetic elements associated with cutaneous Spitzoid tumors. The invention also relates to improved methods for classifying said tumors.

EP 4 112 747 A1

**Description**

**FIELD OF THE INVENTION**

The present invention relates to novel differentially methylated genetic elements associated with cutaneous Spitzoid tumors. The invention also relates to improved methods for classifying said tumors.

**BACKGROUND ART**

Cutaneous Spitzoid tumors include benign Spitz nevus, atypical Spitz tumor (AST) and Spitzoid melanoma. This is a challenging diagnostic group of melanocytic tumors, particularly with regard to the distinction between AST (uncertain malignant potential) and Spitzoid melanoma (malignant) on clinical and histological grounds.

Current diagnostic algorithms are insufficient for optimal clinical and therapeutic management; they are often characterized by a significant lack of objectivity and reproducibility that may lead to false diagnosis. It is thus critical to identify new markers that allow for a reliable and reproducible diagnostic assessment, and that may also be used as a predictive tool in order to anticipate the individualized metastatic potential of each patient, leading to tailored individual therapy. In order to do so, it is also critical to characterize the molecular, genetic and epigenetic signatures of the various types of melanocytic tumors.

The present invention provides an objective and reliable method of classification of Spitzoid tumors, allowing not only to distinguish between benign Spitz nevus and Spitzoid melanoma, but also to subclassify ASTs according to the associated risk of metastasis.

**DETAILED DESCRIPTION OF THE INVENTION**

Cutaneous Spitzoid tumors constitute a heterogeneous subgroup of melanocytic neoplasms with variable malignant potential, ranging from benign to malignant with metastatic dissemination. As stated above, this is a challenging diagnostic group, particularly with regard to the distinction between AST and Spitzoid melanoma on clinical and histological grounds. Thus, predicting the potential metastatic risk of AST and the clinical outcome of the patient poses a major challenge for clinicians.

Different approaches have been used in order to determine the prognosis of ASTs. These approaches include determining characteristic genetic point mutations (HRAS, NRAS, BRAF or BAP1, among others); determining genetic rearrangements that lead to kinase fusion genes (ALK, ROS1, BRAF, NTRK1, RET, MET, NTRK1, NTRK3, MAP3K8, MAP3K3 or PRKCA), causing a downstream activation of Ras-Raf-MEK-ERK-PI3K-Akt and promoting cellular proliferation and migration; determining copy number variations (CNV) of genes, whole chromosomes or subchromosomal regions (e.g., 1q, 3p (3p13, MITF), 6p, 7 (7q23, BRAF), 8q, 11p (11p15, HRAS), 17q, 20q, copy number losses of BAP1 (3p21), 6q, 8p, 9p (9p21, CDKN2A) and 10 (10q23, PTEN)) by comparative genomic hybridization arrays (CGH), FISH or MLPA; immunohistochemical techniques (p16, ki67 or HMB45); and miRNA expression.

Certain approaches combine different techniques and markers, such as immunohistochemistry assays (p16, ki67 and HMB45), FISH (6p25 or 11q13, among others) and CGH arrays or gene expression methods to improve the diagnosis of the tumors.

An emerging approach to determine the prognosis of ASTs is based on epigenetics. Epigenetic alterations are heritable and reversible events that modify gene expression without any change in the DNA sequence, playing an important role in cancer initiation and progression. Epigenetic events include post-translational modifications (PTMs) of histone tails, nucleosome modelling, DNA methylation and non-coding RNAs (short and long), and have been described in the pathological evolution of malignant melanoma.

Whilst the methylation profiles of genes generally affected in melanoma have been studied using the multiple ligand-dependent probe amplification technique (MLPA), these limited methylation studies have not succeeded at identifying relevant genes allowing for the classification of ASTs.

Thus, the diagnostic methods constituting the state of the art are insufficient for optimal clinical and therapeutic management. This lack of accurate diagnostic methods usually leads clinicians to overdiagnose malignancy, and therefore to an unnecessary use of resources. More importantly, overdiagnosis and unnecessary treatment have a severe emotional and physical impact on the patients and their relatives. It is thus essential to identify new markers that allow for a reliable and reproducible diagnostic assessment that may also be used as a predictive tool in order to determine the individualized metastatic potential of each patient, leading to tailored individual therapy.

The present invention relates to a method for classifying a cutaneous Spitzoid tumor in a subject ("method of the invention"). The method allows the classification of cutaneous Spitzoid tumors according to their predicted behaviour as benign Spitz nevus or malignant Spitzoid melanoma depending on/based on the methylation profile of seven different CpG sites. Additionally, the method of the invention allows for the subclassification of ASTs according to their associated metastatic risk i.e., low metastatic risk or high metastatic risk.

Therefore, the method of the invention avoids unnecessary patient examinations and treatment, and allows for tailored patient follow-up, relieving the patient from frequent medical appointments and the associated discomfort and anxiety. Moreover, it also prevents the need for follow-up procedures in those cases where no clinical benefit is likely to be obtained. Importantly, these procedures include the performance of invasive techniques commonly leading to comorbidity, such as sentinel node biopsy and subsequent regional lymphadenectomy; therefore, the method of the invention prevents the exposure of the patient to an unnecessary surgical risk. Finally, a more efficient diagnosis and treatment would lead to significant savings in financial and medical staff as well as in equipment resources.

Thus, the present invention relates to a method for classifying a cutaneous Spitzoid tumor in a subject, said method comprising: a) measuring a level of methylation of at least one CpG site in a biological sample obtained from the subject, wherein the at least one CpG site is differentially methylated in Spitzoid tumors and wherein the tissue sample has been diagnosed as a cutaneous Spitzoid tumor; b) comparing the levels of methylation obtained in a) to two different average reference methylation levels obtained from (i) at least two reference biological samples obtained from at least two subjects diagnosed with a benign Spitz nevus; and (ii) at least two reference biological samples obtained from at least two subjects diagnosed with Spitzoid melanoma; and c) classifying the predicted behaviour of the cutaneous Spitzoid tumor as a benign Spitz nevus or an Spitzoid melanoma, wherein the at least one CpG site is selected from a group consisting of CpG ID NO: 1 to 7.

DNA methylation (5-methylcytosine) is an epigenetic modification that is typically associated with stable transcriptional silencing. This modification plays an important role in several biological processes associated with development and disease, such as cell differentiation, and regulation of gene expression. Additionally, DNA methylation is heavily involved in the development of genetic diseases, carcinogenesis and silencing of intracellular viruses, providing a promising diagnostic tool in medicine. Previous studies to understand the exact role of DNA methylation in certain diseases have led to the characterization of several clinically validated biomarkers, for example MGMT promoter methylation in patients with glioblastoma (PredictMDx, MDxHealth, Inc.); or free-circulating methylated SEPT9 gene copies in plasma as a screening biomarker for colorectal cancer.

The analysis of DNA methylation profiles is typically done through methylation-specific PCR, Methylation Sensitive High-Resolution Melting (MS-HRM), or bisulfite sequencing of DNA. Different techniques may be used to determine and quantify DNA methylation, as described elsewhere in this application.

A CpG site is a DNA region where a cytosine nucleotide is followed by a guanine nucleotide in the sequence of bases along its 5' → 3' direction. Cytosines in CpG dinucleotides can be methylated to form 5-methylcytosines.

CpG sites occur with high frequency in genomic regions called CpG islands (or CG islands). In the present invention, "CpG island" refers to a gene promoter region that contains at least 500 base pairs and a proportion of CG dinucleotides (cytosine-guanine) greater than 50%. Notably, about 70% of promoters located near the transcription start site of a human gene contain a CpG island.

The present inventors have identified a molecular epigenetic signature consisting in seven CpG sites that allow the classification of Spitzoid tumours according to the methylation level of said CpG sites. Thus, the method of the invention allows the classification of cutaneous Spitzoid tumors according to their predicted behaviour as a benign Spitz nevus or an Spitzoid melanoma depending on/based on the methylation profile of these CpG sites, described in Table 1.

**Table 1. Molecular epigenetic signature of the invention.**

| Position (cg) | Gene | NCBI Gene ID | CpG ID NO. |
|---|---|---|---|
| 31149610 | MYO1D | NM_015194.3 | 1 |
| 9826086 | TEKT4P2 | NR_038327.2 | 2 |
| 9825862 | TEKT4P2 | NR_038327.2 | 3 |
| 9825882 | TEKT4P2 | NR_038327.2 | 4 |
| 9826034 | TEKT4P2 | NR_038327.2 | 5 |
| 31149858 | MYO1D | NM_015194.3 | 6 |
| 156186376 | PMF1-BGLAP | NM_001199662.1 | 7 |

The method of the invention comprises measuring a level of methylation of at least one CpG site in a biological sample obtained from the subject and comparing the level of methylation to two different average reference methylation levels, obtained from (i) at least two reference biological samples obtained from at least two subjects diagnosed with a benign Spitz nevus; and (ii) at least two reference biological samples obtained from at least two subjects diagnosed with Spitzoid melanoma

In certain embodiments, the method of the invention comprises measuring a level of methylation of at least one CpG site in a biological sample obtained from the subject and comparing the level of methylation to two different reference methylation levels, wherein the two reference methylation levels are two average values obtained from: (i) at least five reference biological samples obtained from at least five subjects diagnosed with a benign Spitz nevus; and (ii) at least five reference biological samples obtained from at least five subjects diagnosed with Spitzoid melanoma

In certain embodiments, the two reference methylation levels are two average values obtained from: (i) at least ten reference biological samples obtained from at least ten subjects diagnosed with a benign Spitz nevus; and (ii) at least eight reference biological samples obtained from at least eight subjects diagnosed with Spitzoid melanoma

In certain specific embodiments, the two reference methylation levels are two average values obtained from: (i) at least twelve reference biological samples obtained from at least twelve subjects diagnosed with a benign Spitz nevus; and (ii) at least nine reference biological samples obtained from at least nine subjects diagnosed with Spitzoid melanoma In certain embodiments of the invention, the methylation levels of at least one of the seven CpGs in the Spitzoid melanoma reference methylation level are, at least: 5%; 10%; 15%; 20%; 25%, 30%; 35%; or 40% higher than in the benign Spitz nevus average reference methylation level.

In certain specific embodiments of the invention, the methylation levels of at least one of the seven CpGs in the Spitzoid melanoma reference methylation level are, at least 15% higher than in the benign Spitz nevus average reference methylation level.

In certain specific embodiments of the invention, the methylation levels of at least one of the seven CpGs in the Spitzoid melanoma reference methylation level are, at least 20% higher than in the benign Spitz nevus average reference methylation level.

In certain specific embodiments of the invention, the methylation levels of at least one of the seven CpGs in the Spitzoid melanoma reference methylation level are, at least 25% higher than in the benign Spitz nevus average reference methylation level.

In certain embodiments of the invention, the methylation levels of at least two of the seven CpGs in the Spitzoid melanoma reference methylation level are, at least: 5%; 10%; 15%; 20%; 25%, 30%; 35%; or 40% higher than in the benign Spitz nevus average reference methylation level.

In certain specific embodiments of the invention, the methylation levels of at least two of the seven CpGs in the Spitzoid melanoma reference methylation level are, at least 15% higher than in the benign Spitz nevus average reference methylation level.

In certain specific embodiments of the invention, the methylation levels of at least two of the seven CpGs in the Spitzoid melanoma reference methylation level are, at least 20% higher than in the benign Spitz nevus average reference methylation level.

In certain specific embodiments of the invention, the methylation levels of at least two of the seven CpGs in the Spitzoid melanoma reference methylation level are, at least 25% higher than in the benign Spitz nevus average reference methylation level.

In some embodiments of the invention, the at least two of the seven CpGs are CpG ID 1 and CpG ID 2.

In certain embodiments of the invention, the methylation levels of at least three; at least four; at least five; or at least six; of the seven CpGs in the Spitzoid melanoma reference methylation level are, at least: 5%; 10%; 15%; 20%; 25%, 30%; 35%; or 40% higher than in the benign Spitz nevus average reference methylation level.

In certain specific embodiments of the invention, the methylation levels of at least three; at least four; at least five; or at least six; of the seven CpGs in the Spitzoid melanoma reference methylation level are, at least 15% higher than in the benign Spitz nevus average reference methylation level.

In certain specific embodiments of the invention, the methylation levels of at least three; at least four; at least five; or at least six; of the seven CpGs in the Spitzoid melanoma reference methylation level are, at least 20% higher than in the benign Spitz nevus average reference methylation level.

In certain specific embodiments of the invention, the methylation levels of at least three; at least four; at least five; or at least six; of the seven CpGs in the Spitzoid melanoma reference methylation level are, at least 25% higher than in the benign Spitz nevus average reference methylation level.

In some specific embodiments of the invention, the at least three; at least four; at least five; or at least six; of the seven CpGs comprise CpG ID 1 and CpG ID 2.

In certain embodiments of the invention, the methylation levels of the seven CpGs in the Spitzoid melanoma reference methylation level are, at least: 5%; 10%; 15%; 20%; 25%, 30%; 35%; or 40% higher than in the benign Spitz nevus average reference methylation level.

In certain specific embodiments of the invention, the methylation levels of the seven CpGs in the Spitzoid melanoma reference methylation level are, at least 15% higher than in the benign Spitz nevus average reference methylation level.

In certain specific embodiments of the invention, the methylation levels of the seven CpGs in the Spitzoid melanoma reference methylation level are, at least 20% higher than in the benign Spitz nevus average reference methylation level.

In certain specific embodiments of the invention, the methylation levels of the seven CpGs in the Spitzoid melanoma reference methylation level are, at least 25% higher than in the benign Spitz nevus average reference methylation level.

In certain embodiments of the invention, the similarity of the sample from the subject to the reference samples is determined using a statistical or machine learning algorithm ("predictive algorithm" or "predictive model"), including but not limited to, a genetic algorithm, support vestor machine or supervised neural network.

Thus, in some embodiments of the method of the invention, step b) is performed using a predictive statistical or machine learning algorithm.

In some other embodiments of the method of the invention, steps b) and c) are performed using a predictive statistical or machine learning algorithm.

In some specific embodiments of the invention, the predictive algorithm comprises a first equation that calculates the probability of the sample being classified as melanoma, as detailed below ("Equation 1" or "Algorithm 1").

$$P(Melanoma) = \frac{1}{(1 + \exp^{-(-342.3 + 3.4A - 10.3B + 3.4C + 8.9D + 8.0E + 11.0F - 8.7G)})}$$

The indexes "A" to "G" are the percentage of methylation found in each of the seven sites include in the equation (A: CpG ID NO 3, B: CpG ID NO 4, C: CpG ID NO 5, D: CpG ID NO 2, E: CpG ID NO 1, F: CpG ID NO 6, G: CpG ID NO 7).

In some embodiments of the invention, the predictive algorithm comprises a second equation. In some specific embodiments, the second equation comprised in the predictive algorithm is the following equation ("Equation 2" or "Algorithm 2"):

$$P(Melanoma) = \frac{1}{(1 + \exp^{-(-0.16 - 0.012A + 0.014B)})}$$

In some specific embodiments of the invention, the predictive algorithm comprises Equation 1 and Equation 2.

In some specific embodiments of the invention, the predictive algorithm consists of Equation 1 and Equation 2.

As described above, the method of the invention comprises classifying the predicted behaviour of the cutaneous Spitzoid tumor as a benign Spitz nevus or an Spitzoid melanoma (step c).

In certain embodiments of the present invention, a cutaneous Spitzoid tumor may be classified as a benign Spitz nevus present invention if the *P(melanoma)* value obtained with algorithm 1 and/or 2 is < 0.4.

In certain embodiments of the present invention, a cutaneous Spitzoid tumor may be classified as an Spitzoid melanoma if the *P(melanoma)* value obtained with algorithm 1 and/or 2 is ≥ 0.4.

In a second aspect, the present invention relates to a data processing apparatus comprising means for carrying out the method of the invention ("apparatus of the invention").

In certain embodiments, the apparatus of the invention comprises means for carrying out at least step b) of the method of the invention.

In certain embodiments, the apparatus of the invention comprises means for carrying out at least step b) and step c) of the method of the invention.

In certain embodiments, the apparatus of the invention comprises means for carrying out step a), step b) and step c) of the method of the invention.

In a third aspect, the present invention relates to a computer program comprising instructions which, when the program is executed by a computer, cause the computer to carry out the method of the invention ("program of the invention").

The present invention also relates to a computer-readable medium having stored thereon the computer program of the invention.

The term "biological sample" refers to any biological tissues or fluids from a subject, obtained by any method designed for that purpose known to persons skilled in the art. The biological sample comprises DNA, which may or may not be methylated, and may present different degrees of methylation.

As used herein, the term "subject" refers to human and non-human animals, such as veterinary subjects. The term "non-human animal" includes all vertebrates, e.g., mammals and non-mammals, such as non-human primates, mice, rabbits, sheep, dog, cat, horse, cow, chickens, amphibians, and reptiles. In preferred embodiments of the invention, the subject is a human, man or woman of any age or race.

The terms "subject", "individual" and "patient" are used interchangeably in the context of the present invention.

In some embodiments of the invention, the level of methylation is measured at single CpG site resolution.

In some embodiments of the invention, the tissue sample is a Spitz nevus.

The non-specific term nevus relates to visible, circumscribed, chronic lesions of the skin or mucosa. It is applied to several conditions caused by neoplasias and hyperplasias of melanocytes, as well as to a number of pigmentation disorders, both hypermelanotic and hypomelanotic. Nevi can be congenital or acquired; the latter include, but are not limited to, Spitz nevi.

A Spitz nevus is usually >5 mm in diameter, shows a dome or wedge-shaped symmetrical outline with sharp lateral circumscription, maturation, and zonation to the depth of the lesion and epidermal hyperplasia. They do not usually present ulceration, cytonuclear atypia, involvement of subcutaneous tissue or mitoses. The distribution of pigment, if present, may be zonal as well and localized subjacent to the epidermis. On histopathology, Spitz nevi characteristically have vertically arranged nests of nevus cells that have both a spindled and an epithelioid morphology. Kamino bodies may be seen at the dermoepidermal junction.

Treatment of the nevi is usually limited to surgical removal.

In some embodiments of the invention, the tissue sample is a Spitzoid melanoma.

The term Spitzoid melanoma or malignant Spitz tumor (MST) refers to Spitzoid tumors with marked atypia and an aggressive clinical course eventually leading to metastasis and death. Spitzoid melanoma tends to present at a later age than Spitzoid nevi. The skin lesions are larger than those of Spitzoid nevi, often asymmetrical, and with more irregular border and surface. MST is diagnosed on the basis of the following histological criteria: ulceration, necrosis, asymmetrical architecture, infiltrative and/or expansile dermal growth, severe and/ or confluent cytonuclear atypia, dermal mitoses, atypical mitoses, epidermal effacement and peripheral pagetoid extension.

In some embodiments of the invention, the tissue sample is an atypical Spitz tumor (AST).

ASTs share histologic features of both conventional Spitz nevi and spitzoid melanomas, but often follow a favorable clinical course. Historically, they have been described as large tumors comprising densely cellular fascicles of spindled spitzoid melanocytes displaying expansile growth associated with compression of the surrounding stroma. ASTs demonstrate some of the features of a Spitz nevus, such as a spitzoid cytomorphology and epidermal hyperplasia. However, other histologic features overlap with melanoma, including larger size; asymmetry; poor circumscription; ulceration; higher-grade nuclear atypia; aberrant dermal growth; and incomplete or absent maturation.

Whilst an AST may not demonstrate all of these features, it must show enough of them to differentiate it from a Spitz nevus, while not being unequivocally melanoma. ASTs have historically been regarded as having an uncertain biologic potential. They should thus be subclassified into low and high metastatic risk tumors.

In certain embodiments, the method of the invention may comprise classifying the predicted behaviour of an AST sample as a benign Spitz nevus or an Spitzoid melanoma.

Accordingly, in certain embodiments, the method of the invention may comprise subclassifying the predicted behaviour of an AST sample as low metastatic risk and high metastatic risk.

The term "metastasis" relates the spread of cancer from the place where it started in the body to other areas.

In the present invention, the term "low metastatic risk" relates to a low probability of tumor dissemination to distant sites i.e., a predicted behaviour of a benign Spitz nevus *(P(melanoma)* $\geq$ *0.4)*.

In the present invention, the term "high metastatic risk" relates to a high probability of tumor dissemination to distant sites i.e., a predicted behaviour of a benign Spitz nevus *(P(melanoma)* < *0.4)*.

Thus, in those embodiments of the invention where the predictive algorithm is used to determine the similarity of the sample from the subject to the reference sample, and where the algorithm comprises or consist of Equation 1 and/or Equation 2, a higher value of *P* may be associated to a higher metastatic risk.

In some embodiments of the invention, the tissue sample is selected from the group consisting of: a formalin-fixed paraffin-embedded sample; a frozen sample; a fresh sample; and a biopsy or dissected sample.

In some specific embodiments of the invention, the biopsy sample is a liquid biopsy sample.

The method of the invention comprises measuring a level of methylation of at least one of the seven CpG sites of the molecular epigenetic signature. In some embodiments, the method of the invention comprises measuring a level of methylation of one of the seven CpG sites. In some embodiments, the method of the invention comprises measuring a level of methylation of two of the seven CpG sites. In some specific embodiments, the method of the invention comprises measuring a level of methylation of CpG ID NO: 1 y 2.

In some embodiments, the method of the invention comprises measuring a level of methylation of three of the seven CpG sites. In some embodiments, the method of the invention comprises measuring a level of methylation of four of the seven CpG sites. In some embodiments, the method of the invention comprises measuring a level of methylation of five of the seven CpG sites. In some embodiments, the method of the invention comprises measuring a level of methylation of six of the seven CpG sites.

In some embodiments, the method of the invention comprises measuring a level of methylation in CpG ID 1 to 7.

The methods of the invention comprise measuring a level of methylation of at least one CpG site in a tissue sample obtained from the subject. Said measurements may be performed by any method known to persons skilled in the art.

There is a variety of analytical methods for DNA methylation detection, allowing both genome-wide methylation profiling and locus-specific DNA methylation analysis. These methods comprise two major steps. The first one is a methylation-specific reaction or separation, such as bisulfite treatment, methylation specific binding, or methylation specific restriction enzymes. The second major step involves amplification and detection, or direct detection, of the DNA by commonly known techniques inlcuding but not limited to polymerase chain reaction (PCR), Methylation Sensitive High-Resolution Melting (MS-HRM), methylation-specific PCR (MSP), quantitative MSP (QMSP or qMSP), DNA sequencing, mass spectroscopy, Southern blot analysis and restriction enzyme digestion of PCR products amplified from bisulfite- converted DNA.

In some embodiments of the invention, the level of methylation is measured using: a bisulfate conversion-based microarray assay; a differential hybridization assay; a methylated DNA immunoprecipitation based assay; a methylated CpG island recovery assay; a methylation specific polymerase chain reaction assay; a methylation sensitive high resolution melting assay; a microarray assay; a pyrosequencing assay; an invasive cleavage amplification assay; a sequencing by ligation based assay; or a mass spectrometry assay.

Other methods for measuring DNA methylation include restriction landmark genomic scanning, methylation-sensitive representational difference analysis (MS-RDA), bisulfate conversion, padlock probe hybridization, circularization, amplification and next generation or multiplexed sequencing for high throughput detection of methylation.

In some embodiments of the invention, the level of methylation is measured using reduced representation bisulfite sequencing (RRBS).

RRBS is a high-throughput technique developed to analyze genome-wide DNA methylation profiles on a single nucleotide level. The approach combines DNA restriction with enzymes and bisulfite sequencing. Genomic DNA is first digested by a methylation-sensitive restriction enzyme (e.g., BglII, MspI) and size selected to produce a small subset of the genomic DNA enriched for CpG sites in most of the promoters and CpG islands. Bisulfite conversion is performed and sequencing library is constructed subsequently.

Specifically, the method includes the following steps; (i) DNA digestion with a methylation-sensitive enzyme, (ii) end repair and A-tailing, (iii) ligation with methylated sequence adaptors, (iv) fragment purification from agarose gel, (v) bisulfite conversion, (vi) PCR amplification, (vii) PCR product purification and (viii) sequencing.

RBBS may be used on degraded DNA from formalin-fixed paraffin-embedded (FFPE) samples and offers high resolution and coverage of single bases. This approach is often used at early research stages, allowing the identification of differentially methylated regions and their relevance to a given disease.

In some embodiments of the invention, the level of methylation is measured using Methylation-Sensitive High-Resolution melting technology (MS-HRM).

MS-HRM assesses locus-specific DNA methylation and it is optimal for clinical research due to its sensitivity, specificity, high reproducibility and cost-effectiveness.

Unless otherwise defined, all technical and scientific terms used herein have the same meaning as commonly understood by one of ordinary skilled in the art to which this invention belongs. Methods and materials similar or equivalent to those described herein can be used in the practice of the present invention. Throughout the description and claims the word "comprise" and its variations are not intended to exclude other technical features, additives, components, or steps. Additional objects, advantages and features of the invention will become apparent to those skilled in the art upon examination of the description or may be learned by practice of the invention. The following examples and drawings are provided by way of illustration and are not intended to be limiting of the present invention.

## DESCRIPTION OF THE FIGURES

**Figure 1.** PCA from CpG methylation analysis of the three experimental groups.

**Figure 2**. A. Heatmap showing the sites (214) with more than 10% methylation difference with respect to the control (Nevus) (q-value <0.05 when comparing the three experimental groups). B. Heatmap showing the sites (214) with more than 10% methylation difference with respect to the control (Nevus) (q-value <0.05 when comparing the nevus and melanoma groups).

**Figure 3**. A. Heatmap showing the methylation levels of the seven sites from the algorithm 1 (nevus and melanoma groups). B. Heatmap showing the methylation levels of the two sites from the algorithm 2 (nevus and melanoma groups).

**EXAMPLES**

**Materials and methods**

**Human samples.** For this study 40 formalin-fixed paraffin-embedded (FFPE) tumor samples from patients with spitzoid lesions were selected, including 12 Spitz nevi, 19 atypical Spitz tumors (AST) and 9 Spitzoid melanomas (SM) (one of which was a metastatic Spitzoid melanoma). Tumor specimens were collected at the time of surgery, and reported to the Department of Anatomic Pathology of the Hospital Clínico Universitario in Valencia (Spain), from 1999 to 2018. The Ethical and Scientific Committees of the Hospital Clínico Universitario in Valencia approved this protocol. All patients provided written informed consent.

Clinical follow-up, with particular emphasis on the development of metastases and mortality, was recorded, ranging from 24 to 240 months. All tumors were classified according to the 2009 American Joint Committee on Cancer (AJCC) staging system.

**Nucleic acid extraction.** Genomic DNA and total RNA (including small RNAs) from the same tissue section were simultaneously extracted using AllPrep DNA/RNA FFPE (Qiagen), following manufacturer's reccomendations.

Briefly, deparaffinization was performed efficiently using a commercial deparaffinization solution (Qiagen), avoiding further washing steps. After solubilization, both nucleic acids were treated separately to remove formaldehyde crosslinks and then purified automatically using QIAcube nucleic acid purification system (Qiagen). RNA and DNA were eluted using 30 $\mu$l nuclease free water and low EDTA buffer, and stored at -80 and -20 °C, respectively.

Both DNA and RNA were quantified by Nanodrop One (ThermoFhisher Scientific). DNA was isolated for RRBS study, and RNA was extracted for future studies. As required for RRBS, DNA concentration of the samples was also measured using the Qubit dsDNA BR Assay Kit (ThermoFisher Scientific), and the quality was assessed with the Fragment Analyzer and the DNF-487 Standard Sensitivity or the DNF-488 High sensitivity genomic DNA Analysis Kit (Advanced Analytical), according to sample concentration.

**Reduced Representation Bisulfite Sequencing (RRBS).** DNA Methylation Profiling (RRBS Service) (Diagenode Cat# G02020000) was performed. Service workflow included DNA quality control (QC), preparation of RRBS libraries, deep sequencing and primary bioinformatic analysis.

RRBS libraries were prepared using the Premium Reduced Representation Bisulfite Sequencing Kit (Diagenode Cat# C02030033). First, DNA was digested with the restriction enzyme MspI, which recognizes CCGG sites, resulting in genomic fragments which started and ended with a CpG dinucleotide. For the library preparation ends were prepared, adaptors were ligated and samples were selected by size.

Following library preparation, samples were pooled together in groups of eight, and treated with bisulfite. Bisulfite turns non methylated cytosines into uracils, which will be read as timines. Bisulfit treatment was done under minimum DNA degradation conditions and maximum conversion efficacy of non methylated cytosines. Next, library was enriched by amplification via PCR and a clean-up was performed using a 1.45x beads:sample ratio of Agencourt® AMPure® XP (Beckman Coulter).

Qubit dsDNA HS Assay Kit (ThermoFisher Scientific) was used to measure DNA concentration of the pools, and their profiles were checked using the High Sensitivity DNA chip for 2100 Bioanalyzer (Agilent). In case of too high adapter dimer peaks, the pools were size selected one more time using a 1.45x beads:sample ratio of Agencourt®AMPure®XP (Beckman Coulter) and quality control steps were performed again. RRBS library pools were sequenced on a HiSeq3000 (Illumina) using 50 bp single-read sequencing (SR50).

**Bioinformatics analysis.** Quality control of sequencing was performed using FastQC. Trim Galore! version 0.4.1 was used to remove adapters. Then, reads were aligned to the human reference genome hg19 using Bismak v0.16.1, followed by methylation calling using the corresponding Bismark functionality (Krueger and Andrews 2011). These quality controls were performed by the Diagenode RRBS Service, who also provided files as the report with sequencing and CpG methylation statistics, FASTQ files the original row reads, FastQC and trimming reports for the quality of the sequenced reads, alignment files in BAM format, alignment and methylation reports and methylation files of covered Cytosine in bedGraph.

Additionally, we performed other quality control with FastQC and BAM files using three independent programs: Bismark v0.22.1, MultiQC V1.7 and Qualimap v2.2.1. Normalization has been performed with normalizeCoverage function of methylKit R package.

The differential methylation analysis was performed with methylKit R package, applying the Fisher's exact test to obtain p-value and q-value of each site. Predictive models were done by independent logistic regression with the glm function of The R Stats Package.

**Results**

**Differential methylation analysis.** When comparing the methylation status of the nevus, AST (in figure legends:

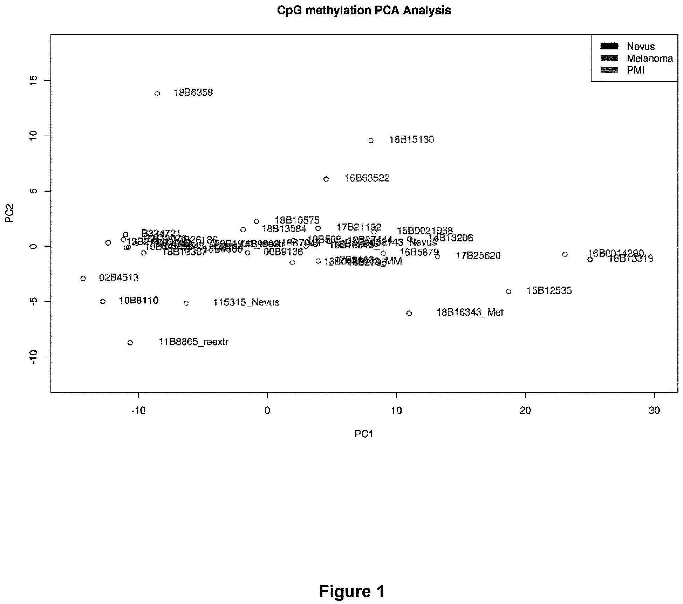

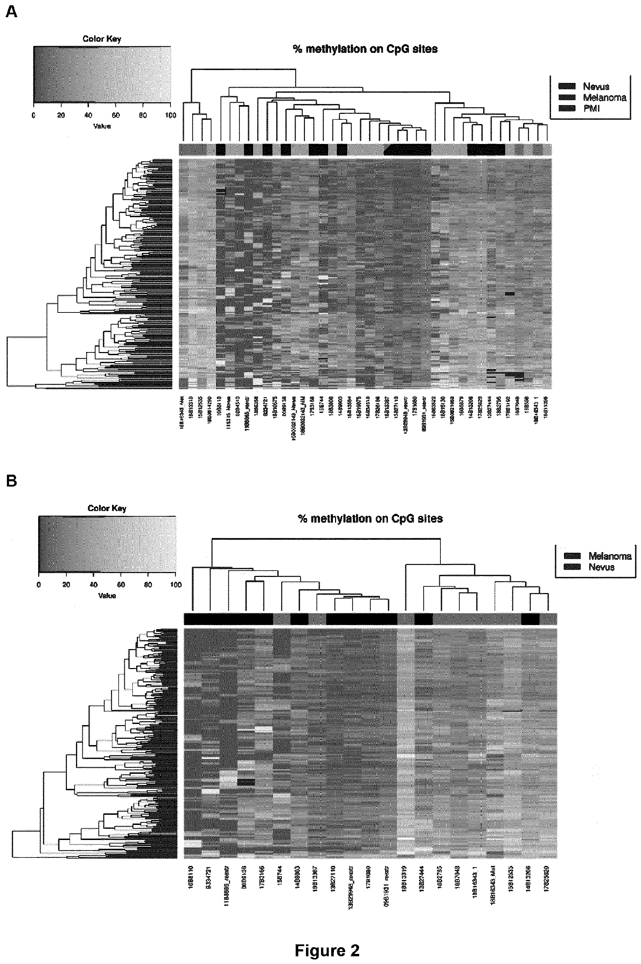

PMI) and SM (in figure legends: Melanoma) samples at the same time, there were no differences between the groups (Figure 1). Only hypermethylated sites (214 sites) were detected in some chromosomes with a significance of q-value <0.05 and methylation difference higher than 10%. The heatmap performed with the significant sites showed that there was no separation between nevus and SM (Figure 2.A) due to the fact that AST includes samples of unknown malignant potential. Studying only the methylation status of the nevi and SM tumors, differences between them were defined (Figure 2.B) and 224 sites were detected, including hypomethylated and hypermethylated.

**Predictive algorithms.** In order to predict the clinical behaviour of the AST, the results obtained from the differential methylation analysis between nevi and SM were used to build the prediction algorithms.

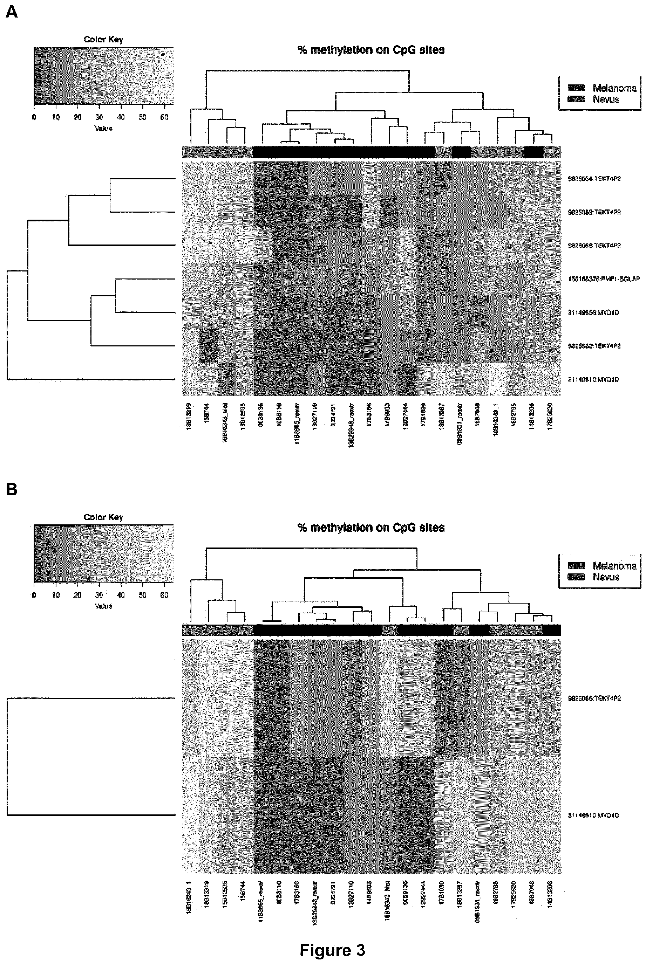

Modelling using binary logistic regression was used for each of the 224 CpG's sites. This analysis resulted in the selection of 7 CpG's (p-value<0,02) for the construction of the predictive algorithms: cg9825862, cg9825882, cg9826034, cg9826086, cg31149610, cg31149858 and cg156186376 (Figure 3.A).

The first predictive algorithm was built with an equation that calculates the probability of being classified as melanoma (Equation or Algorithm 1), where the indices from A to G are the percentage of methylation found in each of the seven sites include in the equation (A: CpG ID NO 3, B: CpG ID NO 4, C: CpG ID NO 5, D: CpG ID NO 2, E: CpG ID NO 1, F: CpG ID NO 6, G: CpG ID NO 7).

$$P(Melanoma) = \frac{1}{(1 + \exp^{-(-342.3+3.4A-10.3B+3.4C+8.9D+8.0E+11.0F-8.7G)})}$$

This algorithm classified correctly all the nevus and melanoma samples with a p-value of 0.0002, allowing the classification of (i) melanoma tumours with a 100% sensibility and (ii) nevus samples with a 100% specificity.

A second prediction algorithm was constructed using the glmulti R package to get the best predictive combination of the seven CpGs, resulting in the CpG ID NO 1 (A) and 2 (B) (Equation or Algorithm 2; Figure 3.B):

$$P(Melanoma) = \frac{1}{(1 + \exp^{-(-0.16-0.012A+0.014B)})}$$

A ROC curve was performed to evaluate the correct melanoma classification power showing an AUC of 0.903 (IC 95% 0.75-1.00) with a p-value of 0.002, obtaining a sensibility of 88.9% and a specificity of 91.7%.

**Prediction of samples.** Regarding the correct classification of the samples used for the construction of the predictive algorithms, Algorithm 1 is capable of classifying 100% of the samples in the correct group (nevus / melanoma) with a p-value of 2e$^{-4}$. In other words, the identified epigenetic signature makes it possible to classify melanoma cases with 100% sensitivity and nevus cases with 100% specificity. Algorithm 2 shows a sensibility of 88.9% and a specificity of 91.7%, with a p-value of 0.002 (Table 1).

**Table 1**. Sample predictions according to the logistic regression algorithms 1 and 2 constructed from 7 differentially methylated CpG sites between Melanoma and Nevus.

| Sample # | Clinically diagnosed Group (AJCC) | Prediction 1 | Prediction 2 |
|---|---|---|---|
| 1 | Melanoma | Melanoma | Melanoma |
| 2 | Melanoma | Melanoma | Melanoma |
| 3 | Melanoma | Melanoma | Nevus |
| 4 | Melanoma | Melanoma | Melanoma |
| 5 | Melanoma | Melanoma | Melanoma |
| 6 | Melanoma | Melanoma | Melanoma |
| 7 | Melanoma | Melanoma | Melanoma |
| 8 | Melanoma | Melanoma | Melanoma |
| 9 | Nevus | Nevus | Nevus |
| 10 | Nevus | Nevus | Nevus |
| 11 | Nevus | Nevus | Nevus |

(continued)

| Sample # | Clinically diagnosed Group (AJCC) | Prediction 1 | Prediction 2 |
|---|---|---|---|
| 12 | Nevus | Nevus | Nevus |
| 13 | Nevus | Nevus | Nevus |
| 14 | Nevus | Nevus | Nevus |
| 15 | Nevus | Nevus | Nevus |
| 16 | Nevus | Nevus | Melanoma |
| 17 | Nevus | Nevus | Nevus |
| 18 | Melanoma | Melanoma | Melanoma |
| 19 | Nevus | Nevus | Nevus |
| 20 | Nevus | Nevus | Nevus |
| 21 | Nevus | Nevus | Nevus |

Regarding the classification of the AST samples for which their real status is unknown, the algorithms classify their behaviour in nevi / melanoma as shown in Table 2.

**Table 2.** Predictions of the borderline AST samples according to the logistic regression algorithms 1 and 2, constructed from 7 differentially methylated CpG sites between melanoma and nevus.

| Sample # | Clinically diagnosed Group (AJCC | Prediction 1 | Prediction 2 |
|---|---|---|---|
| 22 | AST | Nevus | Nevus |
| 23 | AST | Nevus | Nevus |
| 24 | AST | Nevus | Melanoma |
| 25 | AST | Melanoma | Melanoma |
| 26 | AST | Nevus | Nevus |
| 27 | AST | Melanoma | Melanoma |
| 28 | AST | Nevus | Nevus |
| 29 | AST | Melanoma | Melanoma |
| 30 | AST | Melanoma | Melanoma |
| 31 | AST | Melanoma | Melanoma |
| 32 | AST | Nevus | Nevus |
| 33 | AST | Melanoma | Melanoma |
| 34 | AST | Melanoma | Melanoma |
| 35 | AST | Melanoma | Melanoma |
| 36 | AST | Melanoma | Melanoma |
| 37 | AST | Nevus | Nevus |
| 38 | AST | Nevus | Melanoma |
| 39 | AST | Nevus | Melanoma |

Figure 3.A. shows the heatmap of the methylation levels of the seven sites from the algorithm 1 (nevus and melanoma groups). Figure 3.B. shows the heatmap of the methylation levels of the two sites from the algorithm 2 (nevus and melanoma groups).

Considering the classification power of algorithm 1 for the known samples, we conclude that the 2 CpG sites from algorithm 2 explain most of the variance in the predictive model, and the 5 additional CpG sites from algorithm 1 improve the classification.

The obtention of an algorithm with high predictive power for spitzoid lesions according to the methylation status of the seven CpG's of the invention could be a key factor to classify and predict AST behaviour and malignancy individually in a reliable and reproducible manner, and, consequently, to anticipate the metastatic risk of the tumors.

Indeed, the predictive algorithms disclosed herein have high predictive power and offer a solution in the field of personalized precision medicine to the current clinical challenge posed by patients with Spitzoid skin tumors currently classified as "of uncertain malignant potential", thus providing a precise, clear and personalized answer on the diagnosis and prognosis of these patients.

These newly identified epigenetic biomarkers and the associated algorithms could be combined with classical biomarkers, such as immunochemistry assays, FISH or gene expression methods, to improve the diagnosis and the prediction of the evolution of the ambiguous Spitz tumors.

## Claims

1. A method for classifying a cutaneous Spitzoid tumor in a subject, said method comprising:

   a) measuring a level of methylation of at least one CpG site in a biological sample obtained from the subject, wherein the at least one CpG site is differentially methylated in Spitzoid tumors and wherein the tissue sample has been diagnosed as a cutaneous Spitzoid tumor;
   b) comparing the levels of methylation obtained in a) to two different average reference methylation levels obtained from (i) at least two reference biological samples obtained from at least two subjects diagnosed with a benign Spitz nevus; and (ii) at least two reference biological samples obtained from at least two subjects diagnosed with Spitzoid melanoma; and
   c) classifying the predicted behaviour of the cutaneous Spitzoid tumor as a benign Spitz nevus or an Spitzoid melanoma,

   wherein the at least one CpG site is selected from a group consisting of CpG ID NO: 1 a 7.

2. The method of claim 1, wherein the biological sample is a nevus sample.

3. The method of claim 1, wherein the biological sample is a Spitzoid melanoma.

4. The method of claim 1, wherein the biological sample is an atypical Spitz tumor (AST).

5. The method of claim 4, further comprising subclassifying the metastatic risk of the AST sample into low metastatic risk or high metastatic risk.

6. The method of any one of the preceding claims, wherein the biological sample is selected from the group consisting of: a formalin-fixed paraffin-embedded sample; a frozen sample; a fresh tissue sample; a dissected tissue; or a biopsy sample.

7. The method of any one of claims 1 to 6, wherein the level of methylation of the at least one CpG site is measured in CpG ID 1 and CpG ID 2.

8. The method of any one of the preceding claims, wherein the level of methylation of the at least one CpG site is measured in CpG ID 1 to 7.

9. The method of any one of the preceding claims, wherein the level of methylation is measured using: a reduced representation bisulfite sequencing assay; a bisulfate conversion-based microarray assay; a differential hybridization assay; a methylated DNA immunoprecipitation based assay; a methylated CpG island recovery assay; a methylation specific polymerase chain reaction assay; a methylation sensitive high resolution melting assay; a microarray assay; a pyrosequencing assay; an invasive cleavage amplification assay; a sequencing by ligation based assay; or a mass spectrometry assay.

10. The method of any one of the preceding claims, wherein the level of methylation is measured using reduced representation bisulfite sequencing (RBBS).

11. The method of any one of claims 1 to 9, wherein the level of methylation is measured using Methylation-Sensitive High-Resolution melting technology (MS-HRM).

12. The method of any one of the preceding claims, wherein the subject is a human subject.

13. A data processing apparatus comprising means for carrying out the method of according to claim 1.

14. A computer program comprising instructions which, when the program is executed by a computer, cause the computer to carry out the method of claim 1.

15. A computer-readable medium having stored thereon the computer program of claim 14.

CpG methylation PCA Analysis

Figure 1

**A**

**B**

Figure 2

Figure 3

**EUROPEAN SEARCH REPORT**

Europäisches
Patentamt
European
Patent Office
Office européen
des brevets

Application Number

EP 21 38 2569

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | SAINZ-GASPAR L ET AL: "Spitz Nevus and Other Spitzoid Tumors in Children -Part 1: Clinical, Histopathologic, and Immunohistochemical Features", ACTAS DERMOSIFILIOGRAFICAS (ENGLISH EDITION), ELSEVIER, AMSTERDAM, NL, vol. 111, no. 1, 1 January 2020 (2020-01-01), pages 7-19, XP086101654, ISSN: 1578-2190, DOI: 10.1016/J.ADENGL.2019.12.006 [retrieved on 2020-01-17] * page 17 * ----- | 1-6,9-15 | INV. C12Q1/6886 |
| X | CONWAY KATHLEEN ET AL: "Identification of a Robust Methylation Classifier for Cutaneous Melanoma Diagnosis", JOURNAL OF INVESTIGATIVE DERMATOLOGY, vol. 139, no. 6, 1 June 2019 (2019-06-01), pages 1349-1361, XP055863765, NL ISSN: 0022-202X, DOI: 10.1016/j.jid.2018.11.024 * abstract * ----- | 1-6,9-15 | |
| X | WO 2019/144057 A1 (UNIV NORTH CAROLINA CHAPEL HILL [US]) 25 July 2019 (2019-07-25) * paragraph [0168]; claims * ----- | 1-6,9-15 | |

TECHNICAL FIELDS SEARCHED (IPC)

C12Q

-/--

The present search report has been drawn up for all claims

| Place of search | Date of completion of the search | Examiner |
|---|---|---|
| The Hague | 24 November 2021 | Reuter, Uwe |

EPO FORM 1503 03.82 (P04C01)

Europäisches
Patentamt

European
Patent Office

Office européen
des brevets

**EUROPEAN SEARCH REPORT**

Application Number

EP 21 38 2569

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| A | TAKATA M ET AL: "Genetic and epigenetic alterations in the differential diagnosis of malignant melanoma and spitzoid lesion", BRITISH JOURNAL OF DERMATOLOGY, JOHN WILEY, HOBOKEN, USA, vol. 156, no. 6, 1 May 2007 (2007-05-01), pages 1287-1294, XP071117754, ISSN: 0007-0963, DOI: 10.1111/J.1365-2133.2007.07924.X * page 1293 * | 1-15 | |

TECHNICAL FIELDS
SEARCHED     (IPC)

The present search report has been drawn up for all claims

| Place of search | Date of completion of the search | Examiner |
|---|---|---|
| The Hague | 24 November 2021 | Reuter, Uwe |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

## ANNEX TO THE EUROPEAN SEARCH REPORT
## ON EUROPEAN PATENT APPLICATION NO.

EP 21 38 2569

This annex lists the patent family members relating to the patent documents cited in the above-mentioned European search report.
The members are as contained in the European Patent Office EDP file on
The European Patent Office is in no way liable for these particulars which are merely given for the purpose of information.

24-11-2021

| Patent document cited in search report | Publication date | Patent family member(s) | Publication date |
|---|---|---|---|
| WO 2019144057 A1 | 25-07-2019 | NONE | |

For more details about this annex : see Official Journal of the European Patent Office, No. 12/82

EPO FORM P0459